# EUROPEAN PATENT APPLICATION

(11) **EP 1 136 082 A1**
(43) Date of publication of application: **26.09.2001**
(21) Application number: 00201089.0
(22) Date of filing: 24.03.2000
(51) Int. Cl.: A61K 47/48

(54) **Local drug delivery**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Sakharov, Dmitri, 2318 AN Leiden (NL); Emeis, Josephus, Jan, 1018 BE Amsterdam (NL); Rijken, Dingeman Cornelis, 2317 HD Leiden (NL)
(74) Representative: Ottevangers, Sietse Ulbe

(57) **Abstract**

A method for local therapeutic intervention, which provides long-term retention of a highly concentrated drug within the target tissue after local delivery. The invention uses in particular the structural components of tissue as a high capacity carrier/support for accumulation of a high concentration of the drug, providing a rather uniform distribution of the bound drug throughout the tissue. A concentrated drug is to be delivered in the locus of pathology, which drug comprises a therapeutic effector moiety coupled to an affinity vehicle moiety, which affinity vehicle is capable of binding to an abundant structural component of the extracellular matrix of the target tissue, providing long-term retention of the therapeutic effector therein. An important application of the method is in a treatment of vascular disease, particularly restenosis, for which purpose the said drug is to be delivered under pressure into the vessel wall with an appropriate local delivery device.

## Description

### FIELD OF THE INVENTION

The invention is in the field of local drug delivery, more in particular in the field of vascular medicine.

### BACKGROUND OF THE INVENTION

Local drug delivery (LDD) is a useful approach for the treatment of many localized pathologies. The concentration of systemically administered drugs is often limited by side effects of the drug. A much lower amount of drug is required, if the drug can be delivered locally to the site of pathology. Furthermore, a much higher local concentration of drug can be achieved as compared to systemic administration. The efficiency of local drug delivery is often compromised by the fact that the local concentration of the drug may decrease rather soon.

Restenosis is a frequent complication occurring after 30-50% of the percutaneous transluminal coronary angioplasty (PTCA) procedures, increasing mortality and medical costs. No efficient anti-restenotic therapy exists at this moment. A number of potential anti-restenotic drugs has been tested or is currently under investigation including thrombin inhibitors, antiplatelet drugs, ACE inhibitors, anti-growth factor antibodies, anti-integrin antibodies, and cytostatics. No major breakthrough in terms of decrease in incidence of restenosis in humans has been achieved up to now with any of these agents administered systemically. Many of these drugs have been shown to be efficient in animal models, but they failed to bring positive results in humans, probably because the systemic administration does not allow to reach sufficient concentration of the therapeutics during a sufficiently long time period.

Treatment/prevention of restenosis is currently the most tantalizing target for intravascular LDD. The major problem of the intravascular drug delivery is that after being delivered to the site of pathology, the drug is rapidly (in a few minutes) washed out from the site of delivery by the bloodstream. Restenosis is a long process (initial phase takes a few weeks and final changes develop after a few months), and it would be desirable to keep a drug for a long period of time at the site of potential restenosis after local delivery. Incorporation of the drug into microparticles or hydrogels with slow (controlled) drug release is suggested as an approach for prolongation of drug retention (local drug delivery with controlled drug release, LDD-CDR). However, the vessel wall appears to be an anatomic barrier to microparticles delivery; they penetrate poorly into the vascular wall and are found mostly in vasa vasorum and dissections within the intima after angioplasty in an animal model. Drug-containing hydrogel can be incorporated in stents, so that the drug is slowly released from the hydrogel next to the vessel wall. In this technique, the storage of the drug (hydrogel coating) is located next to the target tissue, but not therein; thus, the drug, after being released from the hydrogel compartment, has to penetrate the neighbouring vascular tissue. It is not clear yet what would be the concentration of the drug inside the vessel wall and for how long it would be sustained with this method of delivery.

Besides LDD, another alternative to systemic drug treatment is cell-targeted drug delivery (CTDD). In this approach, the drug, endowed with an affinity to a pathology-specific epitope exposed on the cells in the locus of pathology, is supposed to find the site of pathology and concentrate therein due to binding to these epitopes. Within this approach, most of the efforts is concentrated on the anticancer therapy, since a number of cancer-specific epitopes are identified on various malignant cells. The approach is most attractive for the cases where the location of pathology is not precisely known and the local delivery of drug is not possible. Normally, a highly specific antibody should be generated to provide the specific delivery of the drug to the targeted cell population in the presence of a great excess of the irrelevant tissue epitopes. A variant of this approach is to use a ligand to a cellular receptor as a vehicle for targeting a drug to the cell. In any case, with cell-directed targeting, even if local drug delivery is used, only a small portion of the overall administered drug will bind to the target cells, because cell-associated epitopes (especially pathology-specific ones) represent only a minor fraction of all epitopes present in the tissue. In the blood vessel wall, cells occupy only a small fraction of the volume of the tissue. So, in the case of intravascular local delivery, the major part of the drug will be washed out by the bloodstream. In terms of retention of the drug, which has bound to the target cells, this approach is not much different from the conventional systemic or local drug delivery. Once bound to the cell surface, the drug may be either internalized or shedded from the cellular membrane within hours or, at longest, days.

### SUMMARY OF THE INVENTION

The present invention provides a method of local therapeutic intervention in a pathological process in a soft tissue of animals including humans, comprising local delivery to the site of pathology of a drug comprising a therapeutic effector moiety coupled to an affinity vehicle moiety, wherein said affinity vehicle moiety is capable of binding to a component of the extracellular matrix (ECM) of the target tissue.

The subject invention furthermore provides a pharmaceutical composition comprising a drug and a pharmaceutically acceptable carrier or diluent, wherein the drug comprises a therapeutic effector moiety coupled to an affinity vehicle moiety capable of binding to a component of the extracellular matrix (ECM) of a soft tissue of animals including humans.

The present invention also provides a drug comprising a therapeutic effector moiety coupled to an affinity vehicle moiety, wherein said affinity vehicle moiety is capable of binding to a component of the extracellular matrix (ECM) of a soft tissue of animals including humans.

### DETAILED DESCRIPTION OF THE INVENTION

In the present patent application we disclose a novel method of ECM-targeted local drug delivery (ECM-LDD). We also disclose pharmaceutical compositions and drugs suitable for this method. The method is designed to provide a long-term retention of a highly concentrated drug in the site of local delivery. A principal feature of the approach of the subject invention, which distinguishes it from other drug targeting and local drug delivery systems, is that in particular the structural components of tissue would be used as a high capacity carrier/support for accumulation of a high concentration of the drug, providing a rather uniform distribution of the bound drug throughout the tissue. Unlike the epitopes exposed on cell membranes, the structural epitopes are stable and are present throughout the tissue at a concentration several orders of magnitude higher than that of the cell membrane-associated epitopes. Bifunctional drugs comprising an affinity vehicle moiety and a therapeutic effector moiety will be used. After local delivery, such a drug would be retained within a target tissue due to affinity binding of the affinity vehicle moiety to an abundant and ubiquitous component(s) of the extracellular matrix of the tissue. Some of the potential affinity vehicles used by us concentrate up to a very high concentration of 50 g/L or even 100 g/L within the vessel wall after intravascular delivery and are retained therein for at least day-long periods.

Broadly, this invention can be useful for treatment of a variety of pathologies, which involve a local abnormality in any soft tissue, to which a drug can be delivered locally, either via an intra-tissue injection or via a catheter if the drug has to be delivered to the wall of any hollow "vessel" in the human body. Besides treatment of vascular pathologies, the invention can also be used for local intervention in cancer with vascular access, and drug delivery in any other tubular structures within the human organism, such as urethra, prostate, fallopian tubes etc. Furthermore, the invention can be useful in the cases where the drug can be locally injected with a needle into a site of a local pathology in a soft tissue. In the field of vascular medicine, the invention can be applied for the treatment or prevention of restenosis. In this case, the implementation of the method would require instrumental means for local intravascular delivery of the drug.

An important feature of the approach of this invention is that the affinity vehicle does not have to show a site-specific binding. The local accumulation of the drug will be garantied by the local character of delivery, for which purpose a suitable catheter may be used. For treatment of vascular pathologies, the target within the vessel wall to which the affinity vehicle binds, should preferably be represented by a molecule or a group of molecules abundantly and ubiquitously present in the vessel wall. This will ensure a high concentration and uniform distribution of the delivered drug within the vessel wall. After the delivery step, the drug can either be long-term retained within the tissue, or continuously dissociate therefrom, providing, in the latter case a sort of controlled drug release system.

Herein, "abundant" refers to an occurrence or presence of a target component at concentrations of 0.1 g/L or above. According to this invention, it is preferred that the abundant component is present at a concentration of at least 0.5 g/L, or even more preferably at least 1 g/L, such as in particular at a concentration of 1-100 g/L.

For the intravascular delivery, the affinity vehicles can be chosen from a variety of proteins, peptides and antibodies, known to bind to major components of the ECM of the vascular wall. Alternatively, phage display can be used for selection of suitable peptides capable of strong binding to the vascular tissue. In one of the preferred embodiments, glycosaminoglycans of the ECM can be used as a target for binding of the drugs disclosed herein. It is preferred in this invention that the affinity vehicle has an equilibrium association constant of binding to the target component of at least 10⁶ M⁻¹. Furtheron, such binding is referred to as a "high-affinity binding".

Broadly, the choice of the therapeutic effectors within the suggested approach of ECM-directed affinity targeting is dependent on the concrete pathology to which this approach is applied. In the particular case of restenosis after coronary angioplasty, the therapeutic effector should eventually influence the physiology of vascular smooth muscle cells and/or endothelial cells, through either indirect or direct interaction with the cells. Three processes are believed to contribute to restenosis: 1) vascular remodelling, including early recoil and/or later contraction of the vessel, 2) thrombosis, and 3) smooth muscle cells migration from media to intima, accompanied by their proliferation and extensive ECM synthesis, leading to neointima formation. Therapeutic effectors can be chosen from a variety of substances which affect cellular functions, tissue remodeling or haemostatic/ fibrinolytic system, in particular, anticoagulant and antiplatelet agents, vasorelaxants, cytostatics, inhibitors of smoth muscle cells migration and synthesis, stimulants of re-endothelisation, etc.

As stated above, the invention discloses a new method for local therapeutic intervention, which provides a long-term retention of a highly concentrated drug within the locus of pathology. The major idea of the method is using the structural components of tissue itself as a high capacity carrier/support for accumulation of the drug after local delivery. Drugs, suitable for this approach, are supposed to consist of a therapeutic effector moiety, capable of affecting the pathologic processes within the target tissue, and an affinity vehicle moiety, capable of delivering and retaining the drug within the extracellular matrix (ECM) of the vessel wall. After being delivered to the desired site, such a drug will be then retained therein due to affinity binding to an abundant and ubiquitous component of the extracellular matrix. Importantly, pathology-specific recognition of the target tissue by the affinity vehicle is not required within this approach. Accumulation of the affinity vehicle within the locus of pathology is to be provided by local delivery via an appropriate local delivery device, and by affinity binding to an abundant component of the ECM. Major structural components are present in tissues at very high concentrations (approx. 1-100 g/L); if a proper affinity vehicle would saturate binding sites present on such a component, the concentration of the affinity vehicle bound and retained throughout the target tissue can be equally high (1-100 g/L), several orders of magnituge higher than drug concentration achievable with systemic drug delivery.

Herein, "saturation" refers to an occupation of at least 50% of accessible high affinity binding sites on the target component of ECM by the drug. It is preferred that at least 95% of said accessible high affinity binding sites is occupied by the drug according to this invention.

A number of modern methods of drug delivery are currently being designed with a purpose of surmounting the shortcomings of the systemic drug administration (SDA). Local drug delivery (LDD) can bring a concentrated drug directly to the site of pathology with an appropriate local delivery device, providing high local concentration of a delivered drug unachievable with systemic drug administration. However, the retention of the drug within the target tissue is normally low, especially in the case of intravascular delivery due to wash-out effect of the bloodstream. Local delivery of encapsulated drugs with subsequent controlled drug release (CDR-LDD) provides a somewhat longer retention, but in fact the drug is retained within the polymer (gel or microparticles), and still has to penetrate into the target tissue after being released from the polymer. The distribution of the drug within the target tissue in this case is highly non-uniform, thus, parts of the pathological tissue may remain unaffected by a polymer-encapsulated drug. In cell-targeted drug delivery (CTDD), the drug has to be targeted to pathology-specific epitopes exposed on the cells in the site of pathology. Identifying such epitopes and generation of molecules capable of specifically recognizing these epitopes is a technically difficult task. Even if this problem is resolved, the pathology-specific epitopes are mostly exposed at low concentration, so only a limited amount of the drug can be delivered with this method. The membrane-associated cellular epitopes are relatively labile, so the retention of the drug, delivered to such an epitope, will be limited by active cellular processes like shedding, receptor internalization etc.

The method of ECM-targeted drug delivery (ECM-LDD) disclosed herein is quite different from these methods. Using the tissue as a natural high-capacity matrix/carrier for accumulation of the drug is the essential and new feature of this method, which allows to combine the advantages of the aforementioned methods and to avoid their shortcomings. The major difference of the method from CTDD is that not cellular and pathology-specific epitopes, but rather extracellular structural epitopes of the tissue, which are abundantly and ubiquitously present throughout the tissue, are used as sites for the binding of drug. This allows to reach a very high concentration of the bound drug due to the high concentration of the binding sites in the tissue and a sufficient affinity of the drug to these binding sites. Unlike cell membrane-associated epitopes, these binding sites are stable, which is important for the long-term retention of the delivered drug. Importantly, the drug is concentrated within the tissue, not inside a polymeric support medium, as in the CDR-LDD methodology. Technically, the drug has to be delivered to the site of pathology using LDD instrumentation (catheters, intravascular delivery devices), but the local delivery would result in the case of ECM-LDD in a long-term retention of a large proportion of the drug within the tissue, whereas in the case of conventional LDD the major part of the drug would be washed out within minutes. Depending on the therapeutic rationale, the drug can be retained or released into the tissue in a controlled manner as described below.

The table summarizes some advantages and disadvantages of four variants of drug administration discussed above as compared to the method of the ECM-targeted affinity drug delivery (ECM-LDD), disclosed in this application.

| ADVANTAGES | SDA | CTDD | LDD | CDR-LDD | ECM-LDD |
|---|---|---|---|---|---|
| high concentration in the target | - | +/- | + | +/- | + |
| uniform distribution in the target | + | +/- | + | - | + |
| long-term retention | - | +/- | - | +/- | + |
| absence of systemic side effects | - | + | + | + | + |
| pathology-specific epitope not required | + | - | + | + | + |
| SDA - systemic drug administration | | | | | |
| CTDD - cell-targeted drug delivery | | | | | |
| LDD - local drug delivery | | | | | |
| CDR-LDD - controlled drug release after local drug delivery | | | | | |
| ECM-LDD - extracellular matrix-targeted local drug delivery | | | | | |

Broadly, this invention can be useful for treatment of a variety of pathologies, which involve a local abnormality in any soft tissue, to which a drug can be delivered locally, either via an intra-tissue injection or via a catheter if the drug has to be delivered to the wall of any hollow "vessel" in the human body. The invention is generally applicable to any animal, but use with mammals, humans in particular, is preferred.

Besides treatment of vascular pathologies, discussed below in detail, the invention can be used for local intervention in cancer with vascular access, and drug delivery in any other tubular structures within the human organism, as urethra, prostate, fallopian tubes etc. Furthermore, the invention can be useful in the cases where the drug can be locally injected with a needle into a site of a local pathology in a soft tissue. Besides the vascular bed and tubular structures in general, including blood vessels, urethra, fallopian tubes, etc, other soft tissues to which the invention is applicable include muscular tissue, cartilage, nerve tissue, bone marrow, etc.

In vascular medicine, the invention can be used for treatment of the coronary arteries, peripheral arteries and veins, subjected to atherosclerosis. Prevention of restenosis after coronary angioplasty is seen as one of the major potential applications of the invention. Intravascular implementation of the method of ECM-LDD, proposed in this application, requires instrumental means for the local intravascular delivery of drugs. Various sophisticated catheter devices are now available, suitable for local delivery of a highly concentrated drug right to the vessel wall under certain pressure simultaneously with or immediately after PTCA. The drug can be delivered to the desired site using such a catheter, designed for pressure-driven intravascular drug delivery, in order to provide a better penetration of the drug into the luminal part of the vessel wall. Other physical means such as ultrasound, electromagnetic field etc., can be also used for acceleration of mass transfer in order to enhance binding of the drug throughout the tissue. Catheters with an "open lumen" can be used in order to increase the duration of the delivery step. The invention can be also used for prevention of stenosis in bypass grafts. In this variant, a piece of a graft vessel can be treated ex vivo just before the bypass graft operation with a bifunctional drug according to this invention. In both cases, a luminal layer of the vessel wall will be loaded with the drug bound within this layer due to affinity interaction between its affinity vehicle moiety and the target component within the vessel wall. Peri- or extravascular delivery of the drugs disclosed herein to the sites of vascular pathology is also possible.

An important feature of the present approach is that the affinity vehicle does not have to exhibit a site-specific binding. The local accumulation of the drug will be garantied by the local character of delivery, for which purpose a suitable catheter should be used. The target within the vessel wall, to which the affinity vehicle binds, should be represented by a molecule or a group of molecules abundantly and ubiquitously present in the vessel wall. This will ensure high concentration and uniform distribution of the delivered drug within the vessel wall. Thus, targets for affinity binding should be chosen between the major components of the ECM of the vascular wall, such as glycosaminoglycans, collagen, elastin, laminin, fibronectin etc. Binding of the affinity vehicle to the target component of ECM should be strong enough to provide long-term retention. A dissociation rate constant of the order of magnitude 10⁻⁶ sec⁻¹ or lower will be sufficient to retain the drug in the vessel wall for at least several weeks. High affinity of binding, preferably with an equilibrium association constant not lower that 10⁶ M⁻¹, and high concentration of binding sites in the ECM, preferably not lower than 10⁻⁶ M, will result in accumulation of the locally delivered bifunctional drug within the ECM. If the affinity is high enough, the high-affinity binding sites in the tissue may become saturated with the bound drug. In case of intravascular delivery, this will result in saturation of the high affinity binding sites within a luminal layer of the vessel wall, the depth of the layer being dependent on intraluminal concentration of the drug during delivery, concentration of the binding sites within the tissue, pressure gradient, permeability of the tissue for the drug molecules and duration of local drug administration. Importantly, the drug will be concentrated within this layer up to the concentration of the binding sites present in the tissue, so that a local drug concentration of tens or even hundreds g/L can be reached, if the target molecule in the ECM is present at a high concentration. In this way, the tissue ECM is used as a high capacity carrier/support for accumulation of the drug, with a possibility of either further long-term retention or controlled release of the drug into the microenvironment.

In the Examples section, we show that polycationic peptides (poly-L-lysine, in this case) can be accumulated within a subluminal layer after intravascular delivery up to extremely high concentration of 50 g/L and retain therein for days-long periods of time. From these experiments, the value of 50 g/L can be taken as a rough estimate of the local concentration of a drug which can be delivered into the vessel wall with this method (ECM-LDD).

Examples of suitable target components of the ECM include collagen, elastin, laminin, fibrin, fibronectin, thrombospondin, and others. Antibodies capable of binding to such ECM components, or antibody fragments or derivatives capable of binding such ECM components, are useful as affinity vehicle in this invention.

In addition to the aforementioned possible target components in the ECM, glycosaminoglycans (GAGs) represent a preferred target component within the ECM. They are present throughout the majority of tissues, including vascular tissue, at high concentration, and bear a considerable negative charge. We have found that certain positively charged molecules (see Examples section), bind strongly to the vascular tissue, supposedly to glycosaminoglycans, without causing noticeable side effects in an animal model. Such molecules, including poly-L-lysine, protamine, PAMAM dendrimers, a GAG-binding peptide from vitronectin and others can be used as affinity vehicles within the method disclosed here. Binding of such molecules to the ECM is dependent not only on the positive charge, but also on the size of the molecule and on the charge distribution. For instance, polylysine of a molecular weight of 3,000 does not bind sufficiently strong to the vascular tissue, whereas the GAG-binding peptide from vitronectin with a molecular weight of 1,900 does strongly bind to the vascular tissue, although the net positive charge of the polylysine is much higher. A number of molecules, expected to bind to GAGs on the basis of the data from the literature (for instance, lactoferrin, lipoprotein lipase, antithrombin III, the 1-28 peptide from beta-amyloid protein (Aβ), short peptides containing consensus sequence for GAGs recognition) did not show considerable binding in our screening system.

In general, concrete molecules suitable for the role of the affinity vehicles can be selected from the following categories: 1) antibodies or fragments thereof with an affinity to ECM components; 2) proteins, their fragments, and peptides known to bind to components of the ECM (such as, in particular, polycationic peptides and proteins capable of binding to the negatively charged glycosaminoglycan network within the vascular wall); 3) ECM-binding peptides selected from phage peptide display libraries; 4) molecules of a non-protein nature (for instance synthetic dendrimers) if they show sufficient binding to the ECM.

To obtain peptides or antibodies (or fragments thereof) useful as affinity vehicle in the invention, it is possible to use techniques like phage display with appropriate target material (homogenates, extracts, cell culture-derived ECM, purified components) from the ECM of the tissue in question, in particular tissue of the vascular wall. By using such phage display technique with a target material from human vascular wall tissue, the following peptides VW1 to VW3 were found useful as affinity vehicle:
VW1 = ValProTrpMetGluProAlaTyrGlnArgPheLeu,
VW2 = ThrLeuProTrpLeuGluGluSerTyrTrpArgPro,
VW3 = LeuProTrpLysGluProTyrTyrLeuMetProPro.

Although it is preferred that the affinity vehicle has a high affinity to the target component of the ECM, those which have a moderate affinity are useful to provide a continuous dissociation of the delivered drug from the ECM. The affinity of the affinity vehicle may be adjusted to a desirable level in order to provide an optimal relation between retention and release of the delivered drug.

Broadly, the choice of the therapeutic effectors within the suggested approach of ECM-directed affinity targeting is dependent on the kind of pathology to which this approach is applied. In the particular case of restenosis after coronary angioplasty, the anti-restenotic therapeutic effector should eventually influence the physiology of vascular SMC and/or endothelial cells, through either indirect or direct interaction with the cells. Three processes are believed to contribute to restenosis: 1) vascular remodelling, including early recoil and/or later contraction of the vessel, 2) thrombosis, and 3) smooth muscle cells (SMC) migration from media to intima, accompanied by their proliferation and extensive ECM synthesis, leading to neointima formation. Thrombin generated at the site of vascular injury through the activation of coagulation cascade is believed to be critically involved in the initial phase of restenosis. It stimulates activation of platelets, which produce platelet-derived growth factor, a known stimulator of SMC migration, and induces synthesis of endothelin by endothelial cells, a vasoactive peptide causing long-lasting vasoconstriction through stimulation of SMC. Thrombin can also directly interact with SMC through a specific receptor, stimulating contraction of SMC, their proliferation and synthesis of secondary growth factors. SMC integrins mediate the cellular interactions with the vascular matrix during migration; activation of particular integrins may modify the migratory, proliferative and synthetic activity of the cell.

Indirect interaction of the drugs disclosed herein with the cells may be achieved by interfering into the mechanisms of regulation of cell behaviour by various humoral factors. One of the possibilities of the indirect interaction is inhibition of blood coagulation/thrombin generation and platelet activation, since thrombin and platelet-derived growth factor are known to activate SMC proliferation and migration. For this purpose, anticoagulant molecules like hirudin, thrombomodulin, activated protein C, TFPI, platelet antagonists etc. are candidates for the role of the therapeutic effector.

In particular, thrombomodulin is seen as a promising candidate for the role of therapeutic effector. Upon binding to thrombomodulin, thrombin loses its clotting and thrombin receptor-mediated activities, and is more easily inhibited by antithrombin. After inhibition of thrombomodulin-bound thrombin by antithrombin, the complex thrombin-antithrombin dissociates from thrombomodulin, leaving the thrombomodulin moiety capable of binding the next thrombin molecule. Thus, unlike hirudin, thrombomodulin is not consumed by thrombin and provides a non-stoichiometric inactivation mechanism. Activation of the protein C pathway by thrombin-thrombomodulin complex down-regulates further thrombin generation. Thrombomodulin is an important functional protein normally exposed on endothelium, thus the delivery of thrombomodulin to the site of vascular injury will restore a part of the endothelial function.

Other possibilities of indirect interaction include scavenging the growth factors (PDGF, FGF, TGFbeta etc.), which are known to trigger the SMC activation, or scavenging proteases released from the cell surface, which are believed to be involved in migration of the SMC. Neutralizing antibodies against these growth factors and inhibitors of proteases can be used, respectively, as the therapeutic effectors in these variants.

Direct interaction implies that the ECM-immobilized therapeutic effector has to interact with the cells directly and exert its effect either by the receptor-mediated transmembrane signalling or intracellularly if the therapeutic effector is eventually taken up by the cells. Ligands, which can influence cellular functions (such as contractile status, adhesion, synthesis, proliferation, survival etc) through receptor-mediated signalling, can be used as therapeutic effectors within this approach. The purpose would be to stimulate vasorelaxation (particularly by interfering with nitric oxide synthesis), to reduce cellular proliferation and migration, or even to induce a limited apoptosis. An important variant of the proposed method (ECM-LDD) can be designated as "matrix engineering". The receptor-mediated cell-ECM interactions are shown to strongly influence the cell behaviour. The interaction of cells with components of the ECM is mediated by many cellular receptors, including integrins, which are involved in control of cell behaviour. Certain epitopes present on ECM components and containing characteristic amino acid sequences are involved in these interactions, and can either up- or downregulate proliferation, migration and survival of the cells in the vascular wall. The approach of "matrix engineering" intends changing the matrix composition in order to influence the functional behaviour of the cells. For this, desired "active matrix epitopes" conjugated to an affinity vehicle can be delivered at a high concentration on the existing ECM. As a result, new "active matrix epitopes" will be introduced into the matrix, whereas old "epitopes" would be either masked or outnumbered by the newly introduced "epitopes". RGD-containing peptides (present in many ECM proteins and interact with cellular integrins), anti-integrin antibodies, peptides from the disintegrin family, and YIGSR/SIKVAV peptides (important for interaction of cells with laminin) are seen as promising candidates for the role of therapeutic effectors within the "matrix engineering" approach.

A possible variation of the proposed method (ECM-LDD) involves slow dissociation of the drug from the ECM, to which the drug has been delivered, and further interaction of the dissociated drug with cells or extracellular targets within the tissue. This can be achieved either by using a slowly cleavable (hydrolysable) link between the affinity vehicle and the therapeutic effector moieties, or by using a low-affinity affinity vehicle. A reverse relationship exists between the retention of the drug in the vascular wall and the dissociation of the drug bound. A dissociation rate constant of the affinity vehicle within the range of 10⁻⁴-10⁻⁵ sec⁻¹ will ensure the retention time ranging from hours to days, and the drug will be constantly released into the microenvironment. Since the concentration of the drug originally delivered and bound to the ECM can be rather high (grams per liter), this will provide a sort of "controlled drug release system", alternative to using hydrogels and microparticles for the same purpose. In these "controlled release" variants, the therapeutic effector can interact with the cells either directly or indirectly and can be selected from a broad list of substances considered as potential drugs for microparticle- or hydrogel-mediated slow release systems, namely: anti-thrombotic and anti-mitotic agents, immunosuppressive agents, antisense oligonucleotides, cytoskeletal inhibitors, like taxol and cytochalasin, receptor blockers for contractile agonists, Ca²⁺ - regulators etc. Protease inhibitors (such as aprotinin, TIMPs or others) may also be delivered in this way with a purpose of inhibiting of the proteases, which have been shown to be important for cell migration through the matrix. Molecules, such as VEGF, can be delivered to the site of vascular lesion with a purpose of stimulation of re-endothelisation.

The affinity vehicle moiety and the therapeutic effector moiety can be coupled either directly or through a third carrier molecule, such as dextran, polyethyleneglycol, dendrimer of any type, or other. This can be useful for conjugation of many therapeutic effector molecules per one molecule of affinity vehicle. Furthermore, coupling of more than one affinity vehicle molecule per carrier molecule can increase the affinity of the complex to the target material due to possible multipoint interaction with the ECM. If one-to-one complexes are needed, they may be produced either by methods of chemical cross-linking or by production of chimeric recombinant protein. In the case of chemical cross-linking, unstable (hydrolysable) cross-linking agents can be used in order to provide a continuous release of the therapeutic effector in the microenvironment.

Pharmaceutical formulations containing the therapeutic agent of the present invention can be prepared as solutions suitable for local intra-tissue or intravascular delivery. Generally known carriers, such as buffered saline solution can be used. Stabilizing agents, antioxidants, surfactants, preservatives can be present in the pharmaceutical formulation, if necessary. Another possibility of the pharmaceutical formulation is a lyophilized powder, which has to be dissolved in a proper liquidous carrier prior to use. Such a formulation can contain stabilizers like mannitol, sugars and others.

The following examples describe a number of concrete molecules suitable for the role of the affinity vehicle for the ECM-targeted drug delivery into the human and rat vascular wall, and demonstrate the feasibility of their intravascular delivery and long-term retention.

### EXAMPLE 1.

Selection of potential affinity vehicles from peptide phage display.

Phages capable of binding to homogenates of the vascular wall (intima + media) of human aorta were selected from a library of M13 phages carrying 12-mer random peptides. The binding step was performed in 5-fold diluted human plasma in order to reduce unspecific binding. After 4 rounds of panning with high pH elution (100 mM triethylamine, pH 12), 17 selected clones were tested for binding to fresh-frozen sections of the same aorta. For this, sections were incubated with the culture medium of E. coli infected with the cloned phages, then with a dilution of anti-M13 monoclonal antibody, and finally with a dilution of peroxidase-labeled anti-mouse IgG antibodies with subsequent DAB staining. Of 17 clones tested, 10 appeared to be positive (results not shown). DNA-sequencing has shown that the 10 positive clones contained only 3 distinct amino acid-sequences:
VW1: Val-Pro-Trp-Met-Glu-Pro-Ala-Tyr-Gln-Arg-Phe-Leu-observed 6 times
VW2: Thr-Leu-Pro-Trp-Leu-Glu-Glu-Ser-Tyr-Trp-Arg-Pro-observed 2 times
VW3: Leu-Pro-Trp-Lys-Glu-Pro-Tyr-Tyr-Leu-Met-Pro-Pro-observed 2 times

A strong homology between the three sequences is present (underscored), indicating that the peptides may share a similar binding epitope.

### EXAMPLE 2.

Selection of potential affinity vehicles from known peptides. Binding to cross-sections of human aorta.

This example examines binding of a number of potential affinity vehicles and control molecules to fresh-frozen sections of human aorta. The following molecules were chosen on the basis of data from the literature suggesting that they might potentially bind to various components within the vascular ECM: a peptide with a consensus sequence for binding to glycoasaminoglycans, protamine sulfate, poly-lysine and poly-arginine of various molecular weights, lactoferrin, lipoprotein lipase, antithrombin, a GAG-binding peptide from Abeta-amyloid, a GAG-binding peptide from vitronectin, PAMAM Starburst dendrimer (generation 4), and albumin as a control molecule. The aforementioned molecules were labeled with FITC, incubated with sections of human aorta in human plasma at a concentration of 50 ug/ml for 30 min, and binding was assessed using a fluorescence microscope. Of the tested molecules, protamine sulfate, polylysine ( > 20 KDa), poly-arginine (10 KDa), a GAG-binding peptide from vitronectin, and the PAMAM Starburst dendrimer (generation 4) showed a strong binding. Although the patterns of their staining were not completely identical, all the aforementioned molecules bound basically to all parts of the vessel wall. Polylysine (10 KDa) showed a somewhat weaker binding, whereas poly-arginine (92 KDa) caused aggregation and clotting in plasma. The rest of the molecules tested, namely, the peptide with a consensus sequence for binding to glycoasaminoglycans, lactoferrin, lipoprotein lipase, a GAG-binding peptide from Abeta-amyloid, antithrombin, poly-Lys (3 KDa) and albumin did not bind to the sections.

### EXAMPLE 3.

Delivery and retention of an affinity vehicle within a human umbilical artery wall ex vivo.

One end of a 2 cm piece of human umbilical artery was secured on a thin plastic hollow tube connected to a syringe equipped with a manometer. Air was pumped through the artery in order to destroy the endothelium. The vessel was rinsed with a few ml of human citrated plasma containing 0.5 mg/ml FITC-labeled poly-L-lysine (MW approx. 52 000). The open end of the vessel was ligated, and the same plasma with FITC-poly-L-lysine was pumped in with a syringe and kept under pressure of 0.2 bar for 0.5 h. Then, the ligation was removed and the vessel was washed for 5 min with Tris-buffered saline containing 20 mg/ml BSA. The lower part of the vessel (appr. 0.7 cm long) was cut off and snap-frozen in liquid nitrogen. The remaining part attached to the syringe was washed for 24 h by perfusing 100 ml TBS-BSA (containing sodium azide 0.01%) with a peristaltic pump with a speed of 10 ml/min. Half of the remaining vessel was cut off and snap-frozen in liquid nitrogen. The rest of the vessel was perfused for 48 h more (72 h in total) and snap-frozen afterwards. A similar experiment was performed with FITC-labeled BSA.

Cross-sections (10 µm) prepared from all three parts of the vessel were placed on a subject glass, dried, and watched under a fluorescence microscope. It was found that FITC-poly-L-lysine was accumulated in a sharply visible luminal layer of about 40 µm width, and that the bound material was not washed out noticeably, neither after 24 h, nor after 72 h perfusion. Similar results were obtained with the Starburst PAMAM dendrimer (generation 4) labeled with FITC. In contrast, FITC-BSA did not accumulate significantly in the vessel wall and was completely washed out after 1 h wash.

In order to estimate the local concentration of FITC-poly-L-lysine bound within the subluminal layer, 20 µm cross-sections of the umbilical artery were prepared and the bound FITC-poly-L-lysine was eluted thereof with a 20 mM phosphate buffer, pH 7.4, containing 2 M NaCl. A comparison was made between eluted and not eluted sections of the artery, to which FITC-poly-L-lysine had been delivered earlier and washed afterwards for 24 hours with TBS, as described above. The volume of the area saturated with fluorescence can be estimated as 1.8 nL (the perimeter of the vessel wall is 2.2 mm, the depth of penetration of the labeled molecule into the vessel wall is 0.04 mm, and the thickness of the section is 0.02 mm). Incubation of the section in the buffer containing 2 M NaCl resulted in almost complete elution of the bound fluorescence from the section. About 90 ng of FITC-poly-L-lysine was eluted in this way from a single section, as measured with a fluorometer and calculated using a calibration curve with FITC-poly-L-lysine. With these data, the local concentration of the bound poly-L-lysine within the saturated area can be calculated as 90 ng/1.8 nL = 50 g/L.

This example demonstrates the feasibility of the delivery of a prototype affinity vehicle from the lumen into the vessel wall. The delivery was performed under pressure of 0.2 bar achievable with existing catheter-based intravascular delivery devices. The affinity vehicle (poly-lysine with MW 52,000 in this example) was accumulated in a luminal layer of about 40 µm deep after 30 min incubation in the lumen under pressure in undiluted human plasma. Importantly, the bound material was not noticeably washed out after an extensive 72 hours washing, suggesting that also after weeks-long washing a considerable amount of the delivered molecule would stay bound to the ECM. The control molecule without affinity to the ECM was washed out rapidly. Also important, the local concentration of the bound affinity vehicle was extremely high (50 g/L). This gives an estimate of the local concentration of the drug (therapeutic effector) within the vessel wall, which can be achieved with this method of delivery: approx. 50 g/L.

### EXAMPLE 4.

Coupling of a model enzyme (peroxidase) to an affinity vehicle (poly-L-lysine).

The coupling was performed using the periodate method: peroxidase was oxidized with sodium periodate, gel filtered into saline containing 1 mM sodium acetate, pH 4.0, and incubated with Poly-L-lysine (29 KDa) in a carbonate buffer, pH 9.0 for 1 hour. After 2 h incubation with sodium borohydride at 0.2 mg/ml, the material was dialysed against phosphate buffered saline overnight. The enzymatic activity of peroxidase did not change as a result of conjugation with polylysine, as measured with a chromogenic substrate test.

### EXAMPLE 5.

Delivery and retention of the model enzyme (peroxidase) in the umbilical artery ex vivo.
The pressure-driven intravascular delivery of the peroxidase conjugated with poly-L-lysine (and of a control unconjugated peroxidase) was performed exactly as described in example 3. After the delivery, the vessels were perfused for 1 h with TBS-BSA and frozen. Fresh sections (10 µm) were then stained with a mixture of peroxidase sustrate DAB and H₂O₂. This example shows that a model enzyme (peroxidase, in this case) coupled to the affinity vehicle (poly-L-lysine, 29 KDa, in this case) has been delivered and retained within a luminal layer of the artery, unlike a free enzyme (unconjugated peroxidase) with no affinity to the components of the vascular wall. A strong staining was observed in a luminal layer of the artery treated with the conjugate, but not in an artery treated in a similar way with unconjugated peroxidase. This example proves that a model enzyme (peroxidase, in this case) coupled to an appropriate affinity vehicle (poly-L-lysine 29 KDa, in this case) can be delivered and retained within a luminal layer of the artery.

### EXAMPLE 6.

Delivery and retention of an affinity vehicle in the wall of a rat carotid artery in vivo.

Wistar rats were anaesthetized, a 1 cm segment of a common carotid artery was ligated, and the blood was sucked off with a syringe through a thin plastic tubing. Then the segment was filled in with either FITC-poly-L-lysine (52 KDa) or FITC-albumin, both at a concentration of 1 mg/ml in HEPES-buffered saline and incubated under a slight pressure, imposed manually, for 15 min. Afterwards, the tip of the syringe was removed and the bloodstream was restored. The rats were sacrificed after time intervals ranging from 15 min to 72 hours. The treated segments of the carotid arteries were cut off and snap-frozen. The retained fluorescence was assessed with a fluorescence microscope in 8 µm cross-sections. Some of the sections were fixed with 10% formalin, stained with HPS (haematoxylin-phloxin-saffron), and the morphology was studied with a light microscope. It was found that FITC-poly-L-lysine binds throughout the vessel wall at a high concentration and can be found therein 24 hours as well as 72 hours after restoration of the blood flow. In contrast, FITC-albumin was washed away in less than one hour. The morphology of the vessels after delivery of either FITC-poly-L-lysine or FITC-BSA was examined. No indications of inflammation, thrombosis or immune response have been noticed after either treatment. This example demonstrates the principal feasibility of in vivo delivery of an affinity vehicle with its subsequent retention for at least a few days without causing major side effects.

### EXAMPLE 7.

Synthesis and characterisation of a conjugate of hirudine with polylysine and PAMAM dendrimer.

A heterobifunctional cross-linking agent N-[ε-maleimido caproyloxy]succinimide ester (EMCS) was used for conjugation of poly-L-lysine (29 KDa) with recombinant hirudin (Refludan, Hoechst Marion Roussel). Hirudin was first modified with N-Succinimidyl S-Acetylthioacetate (SATA) in order to introduce protected SH-groups into the hirudin molecule. Incubation of 1 mg/ml hirudin with 1.5 mM SATA resulted in modification of 46% of available amino groups in hirudin, which is equivalent to introduction of about 1.8 protected SH-groups per hirudin molecule. Polylysine was incubated for 1 h with 1mM EMCS in order to introduce the SH-reactive maleimide groups into polylysine. After removal of free EMCS from polylysine solution (gel filtration) and deprotection of SH-groups in SATA-hirudin (incubation with 0.5 M hydroxylamine HCl), the two solutions were mixed at a molar ratio hirudin:polylysine of 10:1 and incubated for 1.5 h at room temperature. Analytical gel filtration showed that about 30% of hirudin was conjugated with polylysine. This means that about 3 hirudin molecules were conjugated to each polylysine molecule.

Essentially the same protocol was used to prepare a conjugate of hirudin with Starburst PAMAM dendrimer (generation 4).

The conjugate hirudin-polylysine was tested for ability to deliver active hirudin to cross-sections of human blood vessels. Cross-sections of a human saphenous vein were incubated with the polylysine-hirudin conjugate at 20 µg/ml for 1 hour, the unbound material was washed away, and the sections were further incubated with TRITC-labeled thrombin at 10 µg/ml. After washing away unbound fluorescence, TRITC-thrombin bound was detected with a fluorescence microscope. In this set-up, the bound TRITC-thrombin was detected in the cross-section pre-treated with the polylysine-hirudin conjugate, but not with unconjugated polylysine, hirudin or a mixture of the two. Binding of TRITC-thrombin to the sections, pre-treated with the conjugate, was greatly reduced if TRITC-thrombin was pre-incubated with an excess of hirudin.

### EXAMPLE 8.

Delivery of the polylysine-hirudin conjugate into a segment of a rat carotid artery in vivo.

The polylysine-hirudin conjugate, prepared as described in EXAMPLE 7, has been delivered under a slight pressure into a segment of a rat carotid artery, using a technique described in EXAMPLE 6, at a concentration 0.2 mg/ml. Rats were sacrificed 1 hour after the procedure, cross-sections of the carotid arteries were prepared and incubated with TRITC-thrombin as described in EXAMPLE 7. After washing unbound fluorescence away, the TRITC-thrombin was found to be bound to the sections of the arteries to which the conjugate had been delivered, but not to the control arteries. This example shows that a potential anti-restenotic drug (hirudin in this case) can be delivered to a segment of an artery in vivo, to retain therein and to exert a functional activity (binding of thrombin, in this case) at the site of local delivery.

### EXAMPLE 9.

Synthesis of conjugate of thrombomodulin with polylysine.

The same method of coupling as described in Example 7 is suitable for conjugation of polylysine with thrombomodulin. Poly-L-lysine (MW 52,000) is modified with EMCS as described. Thrombomodulin is modified with SATA and the SH-groups are deprotected as described. Then the EMCS-modified polylysine is mixed with SATA-modified thrombomodulin at a 1:1 molar ratio in the range of concentration of both components from 1 mg/ml to 5 mg/ml. The conjugate can be separated from the unconjugated components by gel filtration and/or gradient ion-exchange chromatography on CM-sepharose or other ion-exchange media.

Thrombomodulin could be detected in the sections of human aorta pre-treated with the conjugate, by immuno-histological methods using anti-thrombomodulin antibodies. Functional activity of thrombomodulin bound to the sections can be detected by using a protein C activation assay.

### REFERENCES.

Fuster V et al. The three processes leading to post PTCA restenosis: Dependence on the lesion substrate. Thromb. Haemost. 74, 552-9 (1995)

McNamara CA et al. Thrombin and and vascular smooth muscle cell proliferation: Implications for atherosclerosis and restenosis. Semin. Thromb. Haemost. 22, 139-44 (1996)

Lefkovits J and Topol E. Pharmacological approaches for prevention of restenosis after percutaneous coronary intervention. Prog. Cardiovasc. Dis. 40, 141-58 (1997)

Bauters C et al. Prevention of restenosis. Future directions. Trends Cardiovasc. Med. 7, 90-4 (1997)

Brieger D and Topol E. Local drug delivery systems for prevention of restenosis. Cardiovascular Res. 35 405-13 (1997)

Slepian MJ. Polymeric endoluminal gel paving:
therapeutic hydrogel barriers and sustained drug delivery depots for local arterial wall biomanipulation. Semin. Interv. Cardiol. 1, 103-16 (1996)

Wilensky RL et al. Direct intraarterial wal injection of microparticles via a catheter: A potential drug delivery strategy following angioplasty. Am. Heart J. 122, 1136-40 (1991)

## Claims

1. A method of local therapeutic intervention in a pathological process in a soft tissue of animals including humans, comprising local delivery to the site of pathology of a drug comprising a therapeutic effector moiety coupled to an affinity vehicle moiety, wherein said affinity vehicle moiety is capable of binding to a component of the extracellular matrix (ECM) of the target tissue.

2. The method of claim 1, wherein the local therapeutic intervention is in a mammal, in particular a human being.

3. The method of claim 1 or 2, wherein the pathology is localized within the vascular bed.

4. The method of any one of claims 1-3, wherein the pathology is restenosis or potential restenosis, in particular after coronary angioplasty or bypass graft surgery.

5. The method of any one of claims 1-4, wherein the affinity vehicle is a protein or peptide capable of binding to an abundant component, preferably abundant structural component of ECM.

6. The method of any one of claims 1-5, wherein the affinity vehicle has an equilibrium association constant of binding to the target component of at least 10⁶ M⁻¹.

7. The method of any one of claims 1-6, wherein the affinity vehicle is a positively charged molecule capable of interacting with negatively charged components of the ECM, in particular glycosaminoglycans.

8. The method of any one of claims 1-7, wherein the therapeutic effector is a substance affecting cellular functions, tissue remodeling or haemostatic/fibrinolytic system at the site of delivery.

9. The method of any one of claims 1-8, wherein the therapeutic effector is a substance with anti-restenotic activity.

10. The method of any one of claims 1-9, wherein the drug is delivered to the site of vascular pathology extra- or perivascularly.

11. The method of any one of claims 1-10, wherein said drug is locally delivered to the site of pathology at a concentration ranging from 0.1 g/L up to 100 g/L or above, preferably from 1 g/L up to 100 g/L.

12. The method of any one of claims 1-11, wherein said drug is locally delivered to the site of pathology using a device suitable for local intra-tissue delivery.

13. The method of any one of claims 1-12, wherein the target component of ECM is substantially locally saturated with the drug.

14. A pharmaceutical composition comprising a drug and a pharmaceutically acceptable carrier or diluent, wherein the drug comprises a therapeutic effector moiety coupled to an affinity vehicle moiety capable of binding to a component of the extracellular matrix (ECM) of a soft tissue of animals including humans.

15. A drug comprising a therapeutic effector moiety coupled to an affinity vehicle moiety, wherein said affinity vehicle moiety is capable of binding to a component of the extracellular matrix (ECM) of a soft tissue of animals including humans.

16. A substance for local therapeutic intervention in a pathological process in a soft tissue of animals including humans, comprising a therapeutic effector moiety coupled to an affinity vehicle moiety, wherein said affinity vehicle moiety is capable of binding to a component of the extracellular matrix (ECM) of said soft tissue.
